# EUROPEAN PATENT APPLICATION

(11) **EP 1 749 538 A1**
(43) Date of publication of application: **07.02.2007**
(21) Application number: 05727887.1
(22) Date of filing: 30.03.2005
(51) Int. Cl.: A61K 45/00, A61K 35/12, A61K 39/395, A61P 37/02, A61P 37/06, A61P 37/08, A61P 43/00, C07K 16/28, C07K 16/46, C12N 5/06, C12P 21/08, C12Q 1/02, G01N 33/15

(54) **METHODS OF INDUCING DIFFERENTIATION OF REGULATORY T-CELLS AND PROLIFERATING THE SAME WITH GPI ANCHOR PROTEIN AGONIST AND MEDICINAL COMPOSITION THEREFOR**

(30) Priority: 31.03.2004 JP 2004107494; 12.08.2004 JP 2004235272
(71) Applicant: KIRIN BEER KABUSHIKI KAISHA, Tokyo 104-8288 (JP)
(72) Inventor: WATANABE, Tomoko, c/o Pharmaceutical Research Lab., Takasaki-shi, Gunma 3701295 (JP)
(74) Representative: HOFFMANN EITLE
(86) International application number: PCT/JP2005/006119
(87) International publication number: WO 2005/094886

(57) **Abstract**

An object of the present invention is to provide a method for inducing the differentiation of and/or promoting the proliferation of regulatory T cells, an immunosuppressive method using these methods, and pharmaceutical compositions for use in these methods. Furthermore, a method for inducing the differentiation of and/or promoting the proliferation of regulatory T cells and a method for immunosuppression using such methods are provided, which involve causing an agonist of a GIP anchored protein to act on T cells expressing the GIP anchored protein.

## Description

### TECHNICAL FIELD

The present invention relates to a novel method for immunoregulation, particularly to a method for immunoregulation through differentiation induction and proliferation promotion of regulatory T cells (also referred to as suppressive T cells) and relates to a pharmaceutical composition for use in this method.

### BACKGROUND ART

### 1. Regulatory T cells

T cell is one of cell groups that play a central role in the immune system, which is a biophylaxis system against various pathogens. T cells are broadly classified into CD4+ helper T cells and CD8+ cytotoxic T cells. In particular, the former cells can be classified mainly into Th1 cells producing IFN-γ, Th2 cells producing IL-4, and the like based on the cytokine production patterns at specific differentiation and maturation stages after antigen stimulation. The former cells generally contribute to the activation of the cellular immune system and the latter cells generally contribute to the activation of the humoral immune system, both of which are deeply involved in biophylaxis. Immune responses are deeply involved in elimination of pathogens and acquisition of resistance to infection under a delicate balance maintained by the action of these T cells having different properties. It is generally known that in normal immune responses, a mechanism for eliminating foreign non-self antigens functions against such foreign non-self antigens, and such elimination mechanism does not function against self antigens that compose a biological body because of the establishment of immunological tolerance against such self antigens. However, autoimmune disease is caused when excessive immune responses function against self antigens. As described above, immunological tolerance against self antigens is not an absolute mechanism. At the T cell level, various mechanisms by which immunological tolerance is induced are known. One such mechanism is referred to as central tolerance, by which self-reactive T cell clones are eliminated in the thymus gland (Kisielow, P. et al., 1988, Nature 333: 742-746), and the other is referred to as peripheral tolerance, by which self-reactive T cells are controlled outside the thymus gland. Regarding the latter mechanism, cell death induction or anergy induction against self antigens (Rocha, B., and H. von Boehmer, 1991, Science 251: 1225-1228; Jenkins, M. K., and R. H. Schwartz, 1987, J. Exp. Med. 165: 302-319) as well as an active suppression mechanism based on the functions of regulatory T cells (Shevach E. M. 2000, Annu. Rev. Immunol. 18: 423-449) are known. Regulatory T cells are defined as exerting suppressive action against other T cells. Immune responses are established under a delicate balance. For example, it has become known that the above Th1 cells and Th2 cells function antagonistically against their immune responses to each other and that the one acts as a regulatory T cell against the other. At the same time, verification of the presence of regulatory T cell populations and the property analysis of the same are still under much discussion and have been throughout the recent history of immunology. Such regulatory T cells have been studied *in vitro* or *in vivo* as cells having functions to suppress or regulate specific immune responses. Specifically, various cell populations have been reported, which vary in cell surface markers, cytokine profile that are produced by such cell populations, and suppressive and regulatory mechanisms for the production of cytokines (Roncarolo, M. G., and M. K. Levings, 2000, Curr. Opinion. Immunol. 12: 676-683).

Among these regulatory T cells, the cell population that has recently been studied in the most depth is the T cell population with the marker of being CD4+CD25+ as described below. This T cell population has been mainly studied using non-human biological species such as mice and rats. Specifically, it has been revealed that organ-specific autoimmune disease is induced by removing CD25+, RT6.1+ (expressed by most of rat mature T cells), CD5 strongly+, CD45RB weakly+ (mouse), or CD45RC weakly+ (rat) cells from normal mouse or rat CD4+ spleen cells and then transferring the remaining T cells into an immunodeficient animal. Markers specific to such regulatory T cells have not been discovered to date. The above markers cannot be directly correlated with the functions of regulatory T cells and indicate at most a state of cells being activated, a state of cells having been stimulated with an antigen, or a state of cells having immunological memory. However, in addition to such function to induce organ-specific autoimmune disease in an immunodeficient animal, it has become also known that a T cell population that is positive for both CD4 and CD25 can be a regulatory T cell marker, owing to advancement in analyses of regulatory T cell populations using as an index the possession of a function to suppress autoimmune disease and autoimmune inflammation following the transfer of specific cell populations (Sakaguchi, S., et al., 1985, J. Exp. Med. 161: 72; Itoh, M., et al., 1999, J. Immunol. 162: 5317-5326; Sakaguchi. S. et al., 1995, J. Immunol. 155: 1151-1164; Asano, M. et al., 1996, J. Exp. Med. 184: 387-396; Read, S. et al., 2000, J. Exp. Med. 192: 295-302; Salomon, B. et al., 2000, Immunity 12: 431-440; Stephens, L. A., and D. Mason, 2000, J. Immunol. 165: 3105-3110).

As described above, CD4+CD25+ regulatory T cells have been identified in mice and rats. In the meantime, the presence of similar cells in humans was reported by a plurality of groups in 2001 (Jonuleit, H. et al., 2001, J. Exp. Med. 193: 1285-1294; Levings, M. K. et al., 2001, J. Exp. Med. 193: 1295-1301; Dieckmann, D. et al., 2001, J. Exp. Med. 193: 1303-1310; Taama, L. S. et al., 2001, Eur. J. Immunol. 31: 1122-1131; Stephens, L. A. et al., 2001, Eur. J. Immunol. 31: 1247-1245; and Baecher-Allan, C. et al., 2001, J. Immunol. 167: 1245-1253). These reports are based on the fact that the results (obtained through the use of a cell population separated from human peripheral blood using CD4 and CD25 expression, which is known to occur in mice, as an index) in these reports are equivalent to those in reports using mice in terms of various cell surface markers, anergy to stimulation (to cause cell activation), cytokine types that are produced, and general *in vitro* T-cell-proliferation-suppressing functions and the mechanisms thereof, for example. Specifically, CD4+CD25+ T cells separated from human peripheral blood express a CD45RO+ memory T cell marker. Compared with CD4+CD25- T cells, an activation marker such as HLA-DR is expressed at a high level, and CTLA-4 is constantly expressed within cells. The expression level is increased by stimulation. Furthermore, in the case of such CD4+CD25+ regulatory T cells, DNA synthesis and cytokine production are not observed upon stimulation with an anti-CD3 antibody, stimulation with an anti-CD3 antibody and an anti-CD28 antibody, stimulation with allogeneic mature dendritic cells (allogeneic mature DC), or the like. Specifically, CD4+CD25+ regulatory T cells are in an anergy state against antigen stimulation. The DNA synthesis ability of CD4+CD25+ regulatory T cells is enhanced by stimulation with cytokines such as IL-2, IL-4, and IL-15, in addition to an anti-CD3 antibody and an anti-CD28 antibody. However, such enhancement is not equivalent to that in the case of CD4+CD25- T cells. When CD4+CD25- T cells are stimulated with an anti-CD3 antibody or an allogeneic mature dendritic cell (allogeneic mature DC) in the presence of CD4+CD25+ regulatory T cells, the action of suppressing the proliferation of CD4+CD25- T cells is observed depending on the number of CD4+CD25+ regulatory T cells, compared with the case of the absence of CD4+CD25+ regulatory T cells. The facts that have been reported are: CD4+CD25+ regulatory T cells can produce suppressive cytokines such as IL-10 and TGF-β1, the action of suppressing the proliferation of CD4+CD25- T cells is not cancelled with the use of neutralization antibodies against the above cytokines; and the exertion of such suppressive action requires direct cell-to-cell contact between CD4+CD25- T cells and CD4+CD25+ regulatory T cells. The presence of CD4+CD25+ regulatory T cells in mice, rats, and humans has been reported, and the properties thereof are under analyses. No detailed differentiation or suppressive action mechanisms have been elucidated, and no markers specific to such cells have been discovered.

In mice and humans, a regulatory T cell that is induced by stimulation with an allogeneic antigen in the presence of IL-10 or repeated stimulation with an allogeneic immature dendritic cell has also been reported (Groux, H. et al., 1997, Nature 389: 737-742; Jonuleit, H. et al., 2000, J. Exp. Med. 192:1213-1222). Moreover, it has also been reported that regulatory T cells are induced when T cells are simultaneously stimulated with anti-CD3 and anti-CD46, which is known as an entry molecule of certain type of viruses or to suppress complement activation, and when T cells are stimulated using cells forced to express CD58/LFA-3 as an APC (Kemper, C. et al., 2003, Nature 421: 388-392; Wakkach, A. et al., 2001, 167: 3107-3113). These cells produce a large amount of IL-10, unlike Th1 and Th2 cells. However, the cells are characterized by producing a small amount of TGF-β1, IFN-γ, or IL-5, producing IL-2 at a low level, and producing no IL-4. Thus the cells are referred to as Tr1 cells. Tr1 cells are also anergic, similar to the case of CD4+CD25+ regulatory T cells. It may be possible to explain the T-cell-suppressing mechanism of the Tr1 cells based on the fact that they are affected by their own products, including IL-10 and TGF-β1. However, it has not been clarified whether Tr1 cells and CD4+CD25+ regulatory T cells are T cell subsets that are completely different from each other, or whether they are the same cells but differ from each other in terms of differentiation activation stages.

Furthermore, as a new function of 4C8 mAb (Masuyama, J. et al., 1999, J. Exp. Med., 189: 979-990), which is a monoclonal antibody that inhibits the phenomenon whereby human T cells migrate subendothelially after their adhesion to vascular endothelial cells, a regulatory-T-cell-inducing ability has been reported (Masuyama, J. et al., 2002, J. Immunol. 169: 3710-3716; JP Patent Publication (Kokai) 2003-102471). This is based on the fact that cells activated by simultaneous stimulation with an anti-CD3 antibody (OKT3) and 4C8 mAb exert suppressive activity *in vitro* against the proliferation of CD4+ T cells, due to polyclonal stimulation with antibody.

With the use of the expression of CD4 and CD25, which are regulatory T cell markers known in mice and rats, as an index, CD4+CD25+ T cells have also been separated from human peripheral blood and the like. It has been confirmed that CD4+CD25+ T cells separated from human peripheral blood have other cell surface markers and functions similar to those in the same cells of mice or rats. Accordingly, the presence of CD4+CD25+ regulatory T cells in humans has been suggested. These cells are of a scarce cell population, account for only 5% to 10% of peripheral blood CD4+ T cells, and are in an anergy state against activation and proliferation stimulation. In this case, through further stimulation with a cytokine such as IL-2, IL-4, or IL-15 in addition to stimulation with an anti-CD3 antibody and an anti-CD28 antibody, cell proliferation can be promoted. In the case of proliferation promoted by such a technique, cell proliferation cannot be achieved to such a degree that a certain effect is obtained through transfer of such cells into a human. Hence, it is difficult to apply such technique clinically or medically.

The known facts are: that regulatory T cells suppressively function against autoimmune disease (autoimmune encephalomyelitis or colitis) through transfer of the cells into experimental animals (Kohm, A. P. et al., 2002. J. Immunol. 169: 4712-4716; Mottet, C., et al., 2003, J. Immunol. 170: 3939-3943); and that they suppressively function against graft rejection (HvG) and graft versus host disease (GvHD) through transfer thereof into experimental animals (Hara, M. et al., 2001, J. Immunol. 166: 3789-3796; van Maurik, A. et al., 2002, J. Immunol. 169: 5401-5404; Taylor, P. A. et al., 2001, J. Exp. Med. 193: 1311-1317; Taylor, P. A. et al., 2002, Blood 99: 3493-3499; Edinger, M. et al., 2003, Nature Med. 9: 1144-1150; Trenado, A. 2003, J. Clin. Invest. 112: 1688-1696). Therefore, cell therapy using the immunosuppressive action of regulatory T cells has promise as an application for treatment of autoimmune disease and rejection upon organ transplantation. Development of a pharmaceutical composition for promoting the proliferation of regulatory T cells is expected. Alternatively, development of a therapy is expected by which regulatory T cells are proliferated through *ex vivo* treatment of peripheral blood collected from a patient or another human or of bone marrow cells, following which the resultant cells are transferred into the body of a patient.

### 2. GPI-anchored protein

Proteins composing biological membranes include: transmembrane proteins that are present in cell membranes with the use of the hydrophobic portions contained in their own self-amino acid sequences; and proteins modified with lipids, which are bound to the membranes with the use of their lipid (hydrophobic) portions as anchors. GPI-anchored proteins (Glycosylphosphatidylinositol anchored proteins) are of the latter type of protein, where the C-terminal portion of the protein is modified to covalently bind oligosaccharide chain and inositol phospholipid. The GPI-anchored proteins bind to membranes via the lipid hydrophobic portions. 200 or more GPI-anchored proteins have been reported so far, the functions of which also vary widely among differentiation antigens, enzymes, receptors, cell adhesion factors, and the like (Hiroo Ikezawa, 1999, Protein Nucleic Acid and Enzyme 44: 1321-1328). Some GPI-anchored proteins are also expressed by T cells, and the involvement of these GPI anchored proteins in T cell activation has been reported (Horejsi, V. et al., 1998, Immunol. Letter. 63: 63-73; Loertscher, R. and Lavery, P., 2002, Transplant Immunol 9: 93-96). The GPI-anchored proteins reported to be involved in T cell activation are, in the case of mice, Thy-1 (Kroczek, R.A. et al., 1986, J. Immunol 136: 4379-4384), Ly-6 (Malek, T. R. et al., 1986, J. Exp. Med., 164: 709-722), Qa-2 (Hahn, A. B. et al., 1989, J. Immunol. 143: 407-413), and CD48 (Kato, K. et al., 1992, J. Exp. Med. 176: 1241-1249), and in the case of humans, CD52 (Valentin, H. et al., 1992, transplantation. 54: 97-104; Rowan, W.C. 1995, Int. Immunol. 7: 69-77), CD55 (Davis, L.S. et al., 1988, J. Immunol. 141: 2246-2252; Shenoy-Scaria, A. M. et al., 1992, J. Immunol. 149: 3535-3541), CD59 (Korty, P. E. et al., 1991, J. Immunol. 146: 4092-4098), CD73 (Thompson, L. F. et al., 1989, J. Immunol. 143: 1815-1821), BY55/CD160 (Nikolova, M et al., 2002, Int. Immunol. 14: 445-451), and CD48 (Stefanov, I. et al., 1991, Science 254: 1016-1019, Stulnig, T. M. et al., 1997, J. Biol. Chem. 272: 19242-19247), for example. Furthermore, the presence of the GPI-anchored proteins at a specific site on cell membranes that are rich in Sphingolipid/cholesterol (referred to as a lipid raft) has been suggested (Varma, R. and Mayor, S. 1998, Nature 394: 798-801; Friedrichson, T. and Kurzchalia, T. V. 1998, Nature 394: 802-805). It has been reported that the lipid rafts that are present on T cell membranes play an important role in signal transduction of T cell receptors (TCRs) (Xavier, R. et al., 1998, Immunity 8: 723-732; Montixi, C. et al., 1998, EMBO J. 17: 5334-5348; Brdicka, T. et al., 1998, Biochem. Biophys. Res. Commun. 248: 356-360; Kosugi, A. et al., 1999, Int. Immunol. 11: 1395-1401; Zhang, W. et al., 1998, Immunity 9: 239-246; Atsushi Kosugi, 2001, Experimental Medicine (extra edition) 19: 94-101). It is thought that when TCRs on T cells are cross-linked by stimulation with CD3, or the like, cytoskeletal changes are induced and then lipid rafts become accumulated around TCRs on cell membranes. It is thought that such dynamic changes on cell membranes enable TCRs to access signaling factors such as tyrosine kinase belonging to the src family that is present in the intracellular lipid raft domains and thus to perform signal transduction (Horejsi, V. et al., 1999, Immunol. Today 20: 356-361; Simons, K. and Toomre, D. 2000, Nature Reviews Mol. Cell Biol. 1: 31-39; Cherukuri, A. et al., 2001, Immunity 14: 657-660). The way in which the GPI-anchored proteins lacking intracellular domains contribute to T cell activation remains mostly unknown. One possibility is that like TCR-mediated stimulation, this contribution is mediated by various signaling molecules existing in intracellular lipid raft domains where the GPI-anchored proteins are present (Horejsi, V. et al., 1998, Immunology Letters. 63: 63-73; Loertscher, R. and Lavery, P., 2002, Transplant Immunol. 9: 93-96). Furthermore, it is also thought that the GPI-anchored proteins may transmit common signals via lipid rafts, regardless of type (Hale, G., 2001, Cytotherapy 3: 137-143). It has actually been reported that signaling factors to be phosphorylated by cross-linking of the GPI-anchored proteins share a high degree of similarity (Rosemarie, A, et al., 2000, Int. Immunol. 12: 505-516; Tosello, A. et al., 1998, J. Inflamma. 48: 13-27). When the GPI-anchored proteins are cross-linked simultaneously with TCR stimulation, it is highly possible that signals from TCRs are modified. However, induction of regulatory T cells from T cells due to signals mediated by the GPI-anchored proteins has never been reported.

### 3. CD59 antigen

CD59 is an approximately 20-kDa GPI-anchored protein, structurally belonging to the Ly6 family, and expressed in cells of various tissues, including leukocytes. CD59 binds to C9, which is a complement component, thereby inhibiting the binding of C9 to C5b-8, which is the final assembly stage of late complement components. With this inhibition, membrane attack complex formation on cell membranes is suppressed, so as to protect cells from such attack by the complement system. Furthermore, regarding T cells, in addition to the above suppressive action against the complement system, the involvement of CD59 in adhesion through interaction with CD2 is also known (Lovelan, B., 2002, "CD59" in Leukocyte Typing VII, Oxford University Press: 807-809).

Furthermore, in T cell activation, it has been reported that CD59 acts to cause co-stimulation through cross-linking of CD59 antigens with the use of anti-CD59 antibodies upon stimulation with a low dose of PMA (PMA alone is unable to activate T cells), thereby activating and proliferating T cells (Korty, P. E. et al., 1991, J. Immunol. 146: 4092-4098). However, the effects of co-stimulation with CD59 on T cell functions have not yet been clarified.

### 4. CD55 antigen

CD55 is an approximately 70-kDa GPI-anchored protein and is broadly expressed in the tissues of the whole body, including leukocytes. CD55 is known to inhibit C3 convertase formation through binding to C3b and C4b, which are complement components. CD55 is also known to bind to C3bBb and C4b2a so as to promote the lysis of C3 convertase, thereby protecting cells from being damaged by the complement system as a complement control factor (Lovelan, B., 2002, "CD55" in Leukocyte Typing VII, Oxford University Press: 804-805).

Furthermore, regarding T cells, CD55 is also known to act to cause co-stimulation through cross-linking with CD59 antigens like other GPI-anchored proteins do. Upon stimulation with a low dose of PMA (PMA alone at the low dose is unable to activate T cells), cross-linking of CD55 with the use of anti-CD55 antibodies results in proliferation of CD4+ T cells and CD8+ T cells (Davis, L.S. et al., 1988, J. Immunol. 141: 2246-2252). Moreover, the presence of a GPI anchor is essential for co-stimulation mediated by CD55. It is known that in the case of a chimeric CD55 molecule, which is CD55 expressed in the form of being fused with a CD46 transmembrane domain but not with a GPI anchor, co-stimulation due to cross-linking is not observed (Shenoy-Scaria, A. M. et al., 1992, J. Immunol. 149: 3535-3541). However, the effects of co-stimulation mediated by CD55 on T cell functions remain unknown.

### 5. CD48 antigen

CD48 is a 45-kDa GPI-anchored protein and belongs to the immunoglobulin super family. CD48 is broadly expressed by leukocytes. In the case of lymphocytes, enhanced expression of CD48 is observed because of activation. In mice, CD48 is known to be involved in cell adhesion through its binding to CD2. In humans, affinity between CD48 and CD2 is very low. Regarding association of CD48 with disease, it is known that the frequency of CD48 positive lymphocytes is lowered in paraxysmal nocturnal hemoglobinuria patients (Lo, M.F., Sandrin, M., 2002, "CD48" in Leukocyte Typing VII, Oxford University Press: 797-798).

The functions of CD48 (which is expressed by T cells) as a co-stimulation molecule have been particularly examined in mice. The facts that have been reported are: that cross-linking of CD48 with the use of anti-CD48 antibodies upon stimulation with anti-CD3 antibodies results in enhanced T cell proliferation (Kato, K. et al., 1992, J. Exp. Med. 176: 1241-1249); that T-cell-stimulating ability is enhanced in antigen-presenting cells that have been transfected with CD2, the ligand of CD48 (Moran, M. et al., 1998, Immunity 9: 787-796); and that co-stimulation mediated by CD48 through its binding to CD2 is important in induction of cytotoxic T cells (Musgrave, B. L. et al., 2003, J. Interferon Cytokine Res. 23: 67-81), for example. Regarding human T cells, it is also known like other GPI anchored proteins, that cross-linking of CD48 with the use of anti-CD48 antibodies results in enhanced phosphorylation of thyrosin of intracellular signaling proteins (Stefanov, I. et al., 1991, Science 254: 1016-1019) or elevated intracellular calcium concentration (Stulnig, T. M. et al., 1997, J. Biol. Chem. 272: 19242-19247). However, the effects of co-stimulation mediated by CD48 on human T cell functions remain unknown.
- Patent document 1: International Patent Publication No. WO99/12972
- Patent document 2: JP Patent Publication (Kohyo) No. 2-503514 A (1990)
- Non-patent document 1: Masuyama, J. et al., (2002) J. Immunol., 169, 3710
- Non-patent document 2: Hale, G. et al., (1983) Blood, 62, 873
- Non-patent document 3: Xia, M. Q. et al., (1991) European J. Iminunol., 21, 1677
- Non-patent document 4: Kirchhoff, C et al., (1993) Molecular Reproduction and Development, 34, 8
- Non-patent document 5: Pangolis GA et al., (2001) Medical Oncology, 18, 99
- Non-patent document 6: Hederere RA et al., (2000) International Immunology, 12,505
- Non-patent document 7: Valentin H et al., (1992) Transplantation, 54, 97
- Non-patent document 8: Rowan WC et al., (1994) International Immunology, 7, 69
- Non-patent document 9: Kirchhoff C et al., (2001) Cells Tissues Organs, 168, 93

### DISCLOSURE OF THE INVENTION

### Problems to be solved by the invention

An object of the present invention is to provide regulatory T cells having immunoregulatory ability, a method for inducing such regulatory T cells, and a pharmaceutical composition containing such regulatory T cells. Specifically, the present invention relates to a method for inducing the differentiation of and/or promoting the proliferation of regulatory T cells, which is mediated by GPI-anchored proteins including CD59, CD55, and CD48, regulatory T cells induced by such method, and use of such regulatory T cells for autoimmune diseases, allergic diseases, or regulation of immune responses to transplantation.

### Menas to solve the problems

The present inventors have discovered that the differentiation of regulatory T cells is induced and/or the proliferation of the same is promoted with the use of an antibody against CD52 (International Patent Publication No. W02004/087210 (JP Patent Application Nos. 2003-95765 and 2003-413786)). Furthermore, the present inventors have focused on GPI-anchored proteins expressed by T cells while examining the differentiation induction of regulatory T cells. The present inventors have discovered that in T cell activation, antibodies against GPI-anchored proteins (in particular, CD59, CD55, and CD48) that act to cause co-stimulation surprisingly have effects of inducing the differentiation of and/or promoting the proliferation of regulatory T cells through T cell activation, in a manner similar to that in the case of an antibody against CD52.

T cells are activated by co-stimulation mediated by GPI-anchored proteins, so as to contribute to the induction of the differentiation of and/or to promote the proliferation of regulatory T cells. This has been completely unpredictable from knowledge concerning conventional GPI-anchored proteins. Therefore, the present invention represents the first time that the activity of GPI-anchored proteins to induce the differentiation of regulatory T cells has been discovered. This activity can also be useful for immunotherapies for autoimmune diseases, allergic diseases, or regulation of immune responses to transplantation.

The present invention is as follows:
1. a method for inducing the differentiation of a regulatory T cell and/or promoting the proliferation of a regulatory T cell, which comprises stimulating a GPI-anchored protein existing on the surface of an immunocyte other than CD52 with an agonist of the protein;
2. the method according to 1 above, wherein the above GPI-anchored protein is involved in T cell activation;
3. the method according to 1 above, wherein the above GPI-anchored protein is selected from the group consisting of CD55, CD59, and CD48;
4. the method according to 1 above, wherein the above agonist is an anti-GPI-anchored protein antibody or a fragment thereof;
5. the method according to 4 above, wherein the anti-GPI-anchored protein antibody is a humanized antibody or a human antibody;
6. the method according to any one of 1 to 5 above, which further comprises stimulating CD3 existing on the surface of an immunocyte with a CD3 agonist;
7. the method according to 6 above, wherein the CD3 agonist is an anti-CD3 antibody or a fragment thereof;
8. the method according to 7 above, wherein the anti-CD3 antibody is a humanized antibody or a human antibody;
9. the method according to any one of 1 to 8 above, wherein the above immunocyte is a T cell;
10. the method according to 9 above, wherein the above immunocyte is a peripheral blood mononuclear cell;
11. the method according to any one of 1 to 10 above, wherein stimulation of the immunocyte with the CD3 agonist and stimulation of the immunocyte with the GPI-anchored protein agonist are performed *ex vivo;*
12. the method according to any one of 1 to 10 above, wherein stimulation of the immunocyte with the CD3 agonist and stimulation of the immunocyte with the GPI-anchored protein agonist are performed *in vivo;*
13. a regulatory T cell, which is obtained by inducing differentiation and/or promoting proliferation by the method according to any one of 1 to 10 above;
14. a cell-containing product, which contains a regulatory T cell that is obtained by inducing differentiation and/or promoting proliferation by the method according to 1 to 10 above;
15. a pharmaceutical composition for immunosuppression, which contains a regulatory T cell that is obtained by inducing differentiation and/or promoting proliferation by the method according to any one of 1 to 10 above;
16. the pharmaceutical composition according to 15 above for preventing or treating autoimmune diseases, allergic diseases, or immune responses to transplantation;
17. a method for preparing a humanized antibody or an antibody against a GPI-anchored protein other than CD52, which is a drug having effects of inducing the differentiation of a regulatory T cell and/or promoting the proliferation of a regulatory T cell;
18. the method according to 17 above, wherein the GPI-anchored protein is selected from the group consisting of CD55, CD59, and CD48;
19. a method for screening for a drug having the effects of inducing the differentiation of and/or promoting the proliferation of a regulatory T cell using as an index the interaction with a GPI-anchored protein other than CD52, which comprises causing a cell expressing a GPI-anchored protein other than CD52 to come into contact with a candidate compound and then detecting their interaction or a response of the GPI-anchored protein to stimulation;
20. the method according to 19 above, wherein the GPI-anchored protein is selected from the group consisting of CD55, CD59, and CD48;
21. a regulatory T cell derived from a human immunocyte, which is obtained by stimulating an immunocyte collected from a patient's body or another human's body, specifically by stimulating a GPI-anchored protein existing on the surface of the immunocyte, other than CD52, with an agonist of the protein, so as to induce differentiation into a regulatory T cell and to promote the proliferation of the regulatory T cell;
22. the cell according to 21 above, wherein the GPI-anchored protein is selected from the group consisting of CD55, CD59, and CD48;
23. the regulatory T cell according to 21 or 22 above, which is obtained by, in addition to stimulating the above-collected immunocyte with the above agonist, further stimulating the immunocyte with a CD3 agonist, so as to induce the differentiation into a regulatory T cell and promote the proliferation of the regulatory T cell;
24. a method for producing a regulatory T cell derived from a human immunocyte, which comprises stimulating an immunocyte collected from a patient's body or another human's body, specifically by stimulating a GPI-anchored protein existing on the surface of the immunocyte, other than CD52, with an agonist of the protein, so as to induce the differentiation into a regulatory T cell and promote the proliferation of the regulatory T cell;
25. the method according to 24 above, wherein the GPI-anchored protein is selected from the group consisting of CD55, CD59, and CD48;
26. the method for producing a regulatory T cell according to 24 or 25 above, which further comprises stimulating the above-collected immunocyte with a CD3 agonist in addition to stimulating the collected immunocyte with the above agonist;
27. a pharmaceutical composition for inducing the differentiation of and/or promoting the proliferation of a regulatory T cell, which contains an agonist of a GPI-anchored protein other than CD52 as an active ingredient;
28. the pharmaceutical composition according to 27 above, wherein the above GPI-anchored protein is involved in T cell activation;
29. the pharmaceutical composition according to 27 above, wherein the above GPI-anchored protein is selected from the group consisting of CD55, CD59, and CD48;
30. the pharmaceutical composition according to 27 above, wherein the above agonist is an anti-GPI-anchored protein antibody or a fragment thereof;
31. the pharmaceutical composition according to 30 above, wherein the above antibody is a humanized antibody or a human antibody;
32. the pharmaceutical composition according to 27 above, which further contains a CD3 agonist;
33. the pharmaceutical composition according to 32 above, wherein the CD3 agonist is an anti-CD3 antibody or a fragment thereof;
34. the pharmaceutical composition according to 33 above, wherein the anti-CD3 antibody is a humanized antibody or a human antibody; and
35. the pharmaceutical composition according to any one of 27 to 34 above for preventing or treating autoimmune diseases, allergic diseases, or immune responses to transplantation.

### Effects of the Invention

According to the present invention, it has been revealed that T cells that have been activated through co-stimulation mediated by GPI-anchored proteins including CD59, CD55, and CD48 are induced to differentiate into regulatory T cells and/or the proliferation thereof is promoted. These regulatory T cells are useful as active ingredients of pharmaceutical compositions for autoimmune diseases, allergic diseases, and regulation of immune responses to transplantation.

This description includes part or all of the contents as disclosed in the descriptions and/or drawings of Japanese Patent Application Nos. 2004-107494 and 2004-235272, which are priority documents of the present application.

### Brief Description of the Drawings

Fig. 1 shows the results of examining the suppressive effects of regulatory T cells induced by an anti-CD59 antibody on the proliferation of CD4+ T cells resulting from stimulation with an anti-CD3 antibody.
Fig. 2 shows the results of examining the suppressive effects of regulatory T cells induced by an anti-CD55 antibody on the proliferation of CD4+ T cells resulting from stimulation with an anti-CD3 antibody.
Fig. 3 shows the results of examining the suppressive effects of regulatory T cells induced by an anti-CD48 antibody on the proliferation of CD4+ T cells resulting from stimulation with an anti-CD3 antibody.

### Best Mode of Carrying Out the Invention

The present invention relates to the effects of inducing the differentiation of and/or promoting the proliferation of regulatory T cells, which are exerted by an agonist of a GPI-anchored protein, and it relates to an immunosuppressive effect mediated by the regulatory T cells. Hence, the present invention relates to a pharmaceutical composition for the above effects, which contains as an active ingredient an agonist of a GPI-anchored protein, a method for inducing the differentiation of and/or promoting the proliferation of regulatory T cells with the use of such agonist, and a method for immunosuppression mediated by the regulatory T cells. Through stimulation of a GPI-anchored protein existing on the cell surface of an immunocyte (in particular a T cell) with its agonist, differentiation induction of regulatory T cells is caused and the proliferation of the regulatory T cells retaining their functions is promoted. As a result, *in vivo* immunosuppressive effects can be obtained.

In this description, "an agonist of a GPI-anchored protein" is a substance that interacts with the GPI-anchored protein and that can transduce signal into the cell via the protein by stimulating the GPI-anchored protein existing on the surface of an immunocyte. As described above, 200 or more types of GPI-anchored protein have been discovered. Of these GPI-anchored proteins, it has been reported that Thy-1, Ly-6, and Qa-2 are expressed on mouse T cells; that CD52, CD55, CD59, CD73, CD48, BY55/CD160, and the like are expressed on human T cells; and that these GPI-anchored proteins are involved in activation of the cells. Therefore, the GPI-anchored proteins of the present invention may be any GPI-anchored proteins existing on the surfaces of T cells. For use in humans, CD55, CD59, CD73, CD48, and BY55/CD160 are preferable, but the examples are not limited thereto. Signals induced by stimulation with the agonists of GPI-anchored proteins can induce responses including the differentiation of cells into regulatory T cells and/or the proliferation of the regulatory T cells with retaining their properties without change. Examples of such agonists of GPI-anchored proteins; include natural or synthetic ligands of the GPI-anchored proteins, that is, every molecule that induces intracellular signals via a GPI-anchored protein. Furthermore, examples of such agonists also include antibodies against GPI-anchored proteins and fragments thereof. Such antibodies may be any antibodies that recognize any sites of GPI-anchored proteins, as long as they can induce intracellular signals via the GPI-anchored proteins. Examples of such agonists of GPI-anchored proteins also include antibodies against CD55 and CD59 or an antibody against CD48.

An antibody to be used in the present invention may be either a polyclonal antibody or a monoclonal antibody and can be prepared by a method known by persons skilled in the art (e.g., see "New Biochemical Experiment Lecture 1 (Shin Seikagaku Jikken Koza 1), protein I, 389-406, Tokyo Kagaku Dozin Co., Ltd."). Through combination of the steps of evaluating the ability to induce differentiation and/or proliferation of regulatory T cells as described in Examples of the present invention, an antibody to be used in the present invention can be easily obtained. Moreover, commercial antibodies can also be used. Examples of a preferable antibody that is used in the present invention include an anti-CD55 antibody, an anti-CD59 antibody, and an anti-CD48 antibody. Various types of such antibodies are marketed. Examples of such commercially available antibodies include MEM-43 anti-CD59 antibody commercially available from Serotec, anti-CD55 antibody 1C6 clone marketed by Wako, and anti-CD48 antibody MEM102 clone commercially available from Serotec. Furthermore, a so-called humanized antibody that is obtained by transplanting a variable region of an antibody against a GPI-anchored protein into the framework of a human antibody can also be used as an agonist of a GPI-anchored protein of the present invention. Furthermore, a human antibody is obtained with the use of a mouse that retains a non-re-arranged human antibody gene and produces a human antibody specific to the antigen as a result of sensitization with the relevant antigen (e.g., Tomizuka et al., 2000, Proc. Natl. Acad. Sci. U.S.A., 97: 722). The thus obtained human antibody can also be used in the present invention.

In the method of the present invention, the induction of differentiation into regulatory T cells and/or the proliferation of the regulatory T cells can also be promoted by, in addition to stimulating immunocytes expressing CD3 and a GPI-anchored protein with a CD3 agonist, further causing an agonist of a GPI-anchored protein to act on the immunocytes. Specifically, the CD3 signal transduction system that is the main stimulus conducting pathway involved in T cell differentiation is the main stimulation pathway, and a pathway mediated by a GPI-anchored protein is so-called a co-stimulation pathway. In this description, provision of stimulation mediated by a GPI-anchored protein in addition to stimulation mediated by CD3 may also be referred to as co-stimulation with (a) GPI-anchored protein(s). In this description, "CD3 agonist" means any substance that can induce a reaction whereby it acts on a CD3 molecule expressed on the surface of an immunocyte so as to induce signal transduction into the cell via CD3, thereby promoting the differentiation of the relevant cell by signal transduction. Examples of such CD3 agonist include agonistic anti-CD3 antibodies such as OKT3 (ATCC CRL-8001), UCHT1 (B. D. PharMingen), and HIT3a (B. D. PharMingen). Moreover, agonists against various T cell antigen receptors, and in particular, antibodies having agonist action or fragments thereof also cause the formation of complexes of T cell antigen receptors and CD3 and cause signal transduction into the cells via CD3, so that such antibodies or fragments can also be used as CD3 agonists in the present invention. Specifically, another example is OT145 (Posnett et al., 1986, Proc. Natl. Acad. Sci. U. S.A., 83 (20) :7888-92), which is an antibody against the human T cell antigen receptor Vbeta 6.7 or the like. Furthermore, a substance such as a soluble HLA molecule or a tetramer molecule of an HLA molecule and an antigen peptide, which exerts agonist action through recognition by a T cell antigen receptor, can also be used.

As described above, through stimulation of both CD3 and a GPI-anchored protein, the differentiation induction of regulatory T cells is promoted and/or the proliferation of regulatory T cells is promoted. Hence, simultaneous stimulation of CD3 and a GPI-anchored protein may be able to control an excessive immune reaction that takes place *in vivo.* Accordingly, a pharmaceutical composition containing an agonist of a GPI-anchored protein as an active ingredient and a pharmaceutical composition containing the agonist and a CD3 agonist as active ingredients, which are provided according to the present invention, can be useful as immunosuppressive agents. Such pharmaceutical compositions can be useful as drugs targeting the action mechanisms of further specific immune systems; that is, as agents for inducing the differentiation of and/or for promoting the proliferation of regulatory T cells.

Such pharmaceutical compositions that are provided according to the present invention may be compositions to be administered *in vivo.* Alternatively, such pharmaceutical compositions may be compositions for treating *ex vivo* immunocytes collected from a patient or another human, particularly T cells, or peripheral blood, cord blood, bone marrow cells, lymph, lymph node cells, or thymocytes, which contain immunocytes.

The pharmaceutical compositions of the present invention can be formulated by known methods. Specifically, pharmaceutical preparations supplemented with various additives that are acceptable in terms of therapeutic effects, such as a carrier, a pH buffer agent, a stabilizer, and an excipient are produced. Such a pharmaceutical preparation preferably contains a physiologically acceptable diluent or a carrier. Examples of an appropriate carrier include, but are not limited to, a physiological saline solution, phosphate buffered saline, a phosphate buffered saline glucose solution, and a buffered physiological saline. Alternatively, agonists of GPI-anchored proteins are provided in the state of being lyophilized (freeze-dried) and may be reconsitituted through addition of the above aqueous buffered solution and then used, when necessary. Examples of the routes for administration of the above pharmaceutical preparations include peroral administration using tablets, capsules, granules, powders, syrups, or the like and parenteral administration using injections, drops, suppositories, or the like.

The method for administration of and the dose of the above pharmaceutical composition can be appropriately determined during the process of a preclinical test or a clinical test. For example, in the case of peroral administration, the dose of such pharmaceutical composition for an adult is generally approximately between 0.01 mg and 1000 mg per day, but it differs depending on symptom, age, body weight, and the like. Such dose can be administered once or administered at several separate times. Moreover, in the case of parenteral administration, the dose of such pharmaceutical composition is between approximately 0.01 mg and 1000 mg per single administration, and it can be administered through subcutaneous injection, intramuscular injection, or intravenous injection.

Diseases to be treated according to the present invention are diseases that require treatment that provides immunosuppressive effects. Specific examples of such diseases include immune responses to transplantation, such as graft versus host disease (GvHD) or graft rejection and autoimmune diseases such as allergic diseases and rheumatism. For the purpose of treating or preventing graft versus host disease or graft rejection, the pharmaceutical compositions of the present invention and the methods of the present invention can also be used for treatment before and after organ or cell transplantation.

In the present invention, a therapy that can also be employed involves: treating *ex vivo* immunocytes collected from a patient or another human who requires the above treatment or prevention or treating peripheral blood, cord blood, bone marrow cells, lymph, lymph node cells, or thymocytes, which contain the immunocytes, with the use of an agonist of a GPI-anchored protein or if necessary treating such immunocytes with the agonist and a CD3 agonist, so as to cause the proliferation of regulatory T cells, and returning the cells that have proliferated into the patient's body.

Regarding conditions for stimulation with an agonist of such immunocyte, when intracellular signal transduction mediated by a GPI-anchored protein is carried out using a GPI-anchored protein agonist and stimulation with a CD3 agonist is also carried out, such stimulation (mediated by a CD3 agonist) may be carried out using each agonist in an amount sufficient for CD3-mediated intracellular signal transduction to take place. Such amount differs depending on an agonist type. The appropriate amount of an agonist to be used and the time required for agonist stimulation can be determined by persons skilled in the art through a simple test. The time required for stimulation is generally 12 hours or longer, more preferably 24 hours or longer, and particularly preferably about 3 days, for example.

On the condition that the regulatory T cells are returned to the patient from which the cells have been collected or to another human, the present invention thus also encompasses a method for differentiating and proliferating regulatory T cells *ex vivo* according to the above method and regulatory T cells obtained by such procedures. According to the method of the present invention, regulatory T cells derived from one individual (that is, regulatory T cells having a uniform hereditary character) can be obtained in a therapeutically effective amount, although it has been difficult to obtain such amount of regulatory T cells according to conventional technology.

Stem cell transplantation therapy that involves collecting peripheral blood or bone marrow cells from a living body and then returning the cells into a patient's body has already been performed. Furthermore, cancer therapy that involves artificially treating dendritic cells that are of a type of immunocyte and then returning the treated cells into a patient's body has been performed (M. Jefford, et al., The Lancet Oncology, 2: 343-353, June, 2001). An agonist of a GPI-anchored protein and a CD3 agonist are caused to act on collected immunocytes, so that differentiation induction and/or proliferation of regulatory T cells can be promoted. The regulatory T cells are proliferated and then returned to a patient's body, so that therapeutic or preventive effects can be exerted. Implementation of such so-called an *ex vivo* method can also be said to be direct reproduction of an experimental system that has been developed at the site of basic research at a therapeutic site. Compared with *in vivo* administration of a drug with which expected therapeutic effects cannot always be exerted because of the effects of systemic absorption, metabolism, interferential action due to unknown factors, or the like, such an *ex vivo* method has lower risk in practical application.

Furthermore, for the development of a drug having effects of inducing the differentiation of and/or promoting the proliferation of regulatory T cells, the present invention also encompasses causing cells expressing a GPI-anchored protein to come into contact with candidate compounds, detecting the interaction between the protein and such compounds or responses of the GPI-anchored protein to stimulation, and then screening for a compound that can be an agonist of the GPI-anchored protein using the interaction with the GPI-anchored protein as an index. "Responses of a GPI-anchored protein to stimulation " may be activity of inducing the differentiation of regulatory T cells or activity of promoting the proliferation of regulatory T cells, for example.

### EXAMPLES

### Example 1 : Induction of regulatory T cells by co-stimulation with anti-CD59 antibody

It was examined whether regulatory T cells were induced from CD4+ T cells by co-stimulation with a monoclonal antibody, the antigen of which was CD59.

Antibodies were immobilized onto a plate as described below. An anti-CD3 antibody (ORTHOCLONE OKT3, Ortho Biotech) prepared at a concentration of 100 ng/ml in phosphate buffered saline (PBS) was dispensed in a 48-well plate (Costar) followed by 24 hours of incubation at 4°C, so that the antibody was immobilized on the plate. An anti-CD59 antibody (MEM-43, Serotec) prepared at 30 µg/ml in PBS was further immobilized in a manner similar to the above method. Peripheral blood mononuclear cells were separated from the peripheral blood of a healthy volunteer by a density gradient centrifugation method using Ficoll-Paque Plus (Amersham Pharmacia). CD4+ T cells were prepared from the peripheral blood mononuclear cells by negative selection using MACS CD4+ T Cell Isolation Kit (Miltenyi Biotec) according to the reagent manufacturer's instructions. The thus obtained CD4+ T cells were suspended in a RPMI 1640 medium (GIBCO) supplemented with 10% FCS and 15 mM HEPES buffer. The cells were inoculated at 8 x 10⁵ cells/well on the plate on which the above anti-CD3 antibody and the anti-CD59 antibody had been immobilized. The cells were cultured in a CO₂ incubator at 37°C under a 5% CO₂ environment for 3 days. On day 3 of culture, the cells were harvested, washed, and then re-suspended in a medium. The cells were then inoculated in a 24-well plate (Costar) at 1 x 10⁶ cells/well. The cells were then cultured for 4 days with no stimulation, thereby causing the cells to be in a resting state. Subsequently, the cells as the cells that had been co-stimulated with the anti-CD59 antibody were subjected to suppression assay. As a result of co-stimulation with the anti-CD59 antibody, 90% or more of the cells was activated by day 3 of culture to be CD25 positive. Furthermore, the number of the cells on day 7 of culture had increased to about 1.5 times that at the time of inoculation.

Suppression assay was carried out as follows. CD4+ T cells (1 x 10⁵ cells/well) were inoculated with peripheral blood mononuclear cells (4 x 10⁵ cells/well) irradiated with X-ray (5,000 rads) in a 96-well U-bottomed plate (ICN). After addition of an anti-CD3 antibody at a final concentration of 25 ng/ml, the cells were cultured for 3 days. At this time, [³H] thymidine incorporation of a control group to which no regulatory T cells had been added was compared with that of a group to which the cells (co-stimulated with the anti-CD59 antibody and irradiated with X-ray (5,000 rads)) as regulatory T cells had been added at 1 to 2 x 10⁵ cells/well. Suppressive activity was thus evaluated. [³H] thymidine incorporation was evaluated by adding [³H] thymidine at 0.2 µCi/well on day 3 of culture, harvesting cells 8 hours after the addition, and then measuring the count of [³H] thymidine that had been incorporated by the cells using a Beta plate (PerkinElmer). All cells used in a single assay had been derived from the same donor.

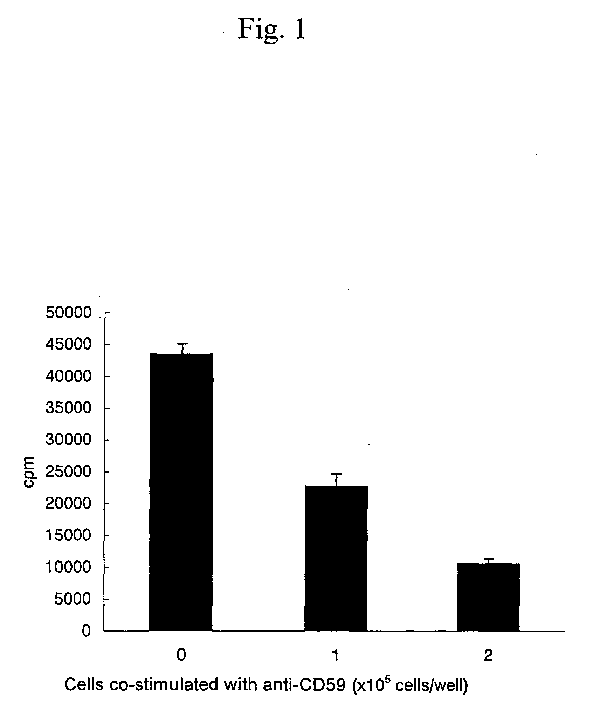

As shown in Fig. 1, the cells co-stimulated with the anti-CD59 antibody suppressed the proliferation of CD4+ T cells due to stimulation with the anti-CD3 antibody, depending on the number of co-stimulated cells added. Thus, it was revealed that regulatory T cells are induced by co-stimulation with the anti-CD59 antibody.

### Example 2 : Induction of regulatory T cells by co-stimulation with anti-CD55 antibody

It was examined whether regulatory T cells were induced from CD4+ T cells by co-stimulation with a monoclonal antibody, the antigen of which was CD55.

Preparation of co-stimulated cells and suppression assay were carried out by the methods described in Example 1. However, an anti-CD55 antibody (clone 1C6, Wako) was used instead of the anti-CD59 antibody. The anti-CD55 antibody used herein had low co-stimulation ability so that it could activate only some of the cells through co-stimulation. Accordingly, the cells were divided into activated cells and non-activated cells using FACS Vantage SE (Becton Dickinson) and CD25 expression as an index. Suppression assay was carried out for each type of cell.

The cells were divided into activated cells and non-activated cells as described below. CD4+ T cells were cultured on a plate on which the anti-CD3 antibody and the anti-CD55 antibody had been immobilized according to the methods described in Example 1. The cells were harvested and washed on day 3 of culture. FITC-labeled anti-CD25 antibody (BD Pharmingen) was added to the cells in a phosphate buffer containing 2% autologous plasma and 2 mM EDTA, followed by 30 minutes of reaction at 4°C. Subsequently, the cells were washed, unbound antibodies were removed, and then the cells were suspended in a phosphate buffer containing 2% autologous plasma and 2 mM EDTA. A 1% 7-AAD solution (BD Pharmingen) was added and then dead cells were stained. Of 7-AAD-negative viable cells, a CD25-FITC-positive cell group and a non-positive cell group were separated and harvested using FACS Vantage SE (Becton Dickinson). The purity obtained using as an index the proportion of CD25+ in the CD25+ cell group after harvest was 94% or higher. Subsequently, the cells following separation were cultured with no stimulation for 4 days in a manner similar to that in Example 1 and then used for suppression assay. Suppression assay was carried out by the methods described in Example 1.

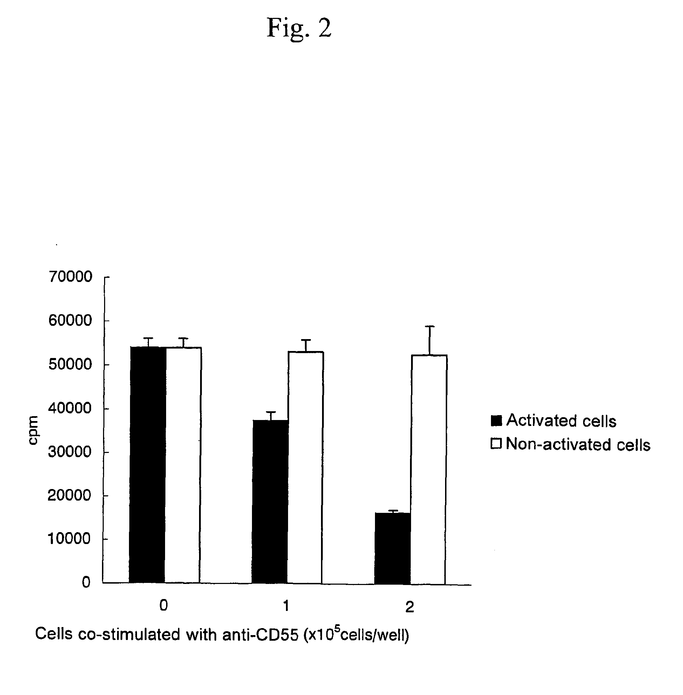

As shown in Fig. 2, among cells co-stimulated with the anti-CD55 antibody, no suppressive activity was observed in the case of CD25- non-activated cells. However, CD25+ activated cells suppressed the proliferation of CD4+ T cells due to stimulation with the anti-CD3 antibody, depending on the number of activated cells. It was revealed that the cells activated by co-stimulation with the anti-CD55 antibody acquire the properties of regulatory T cells.

### Example 3 : Induction of regulatory T cells by co-stimulation with anti-CD48 antibody

It was examined whether regulatory T cells were induced from CD4+ T cells by co-stimulation with a monoclonal antibody, the antigen of which was CD48.

Preparation of co-stimulated cells and suppression assay were carried out by the methods described in Example 1. However, an anti-CD48 antibody (clone MEM102, Serotec) was used instead of the anti-CD59 antibody.

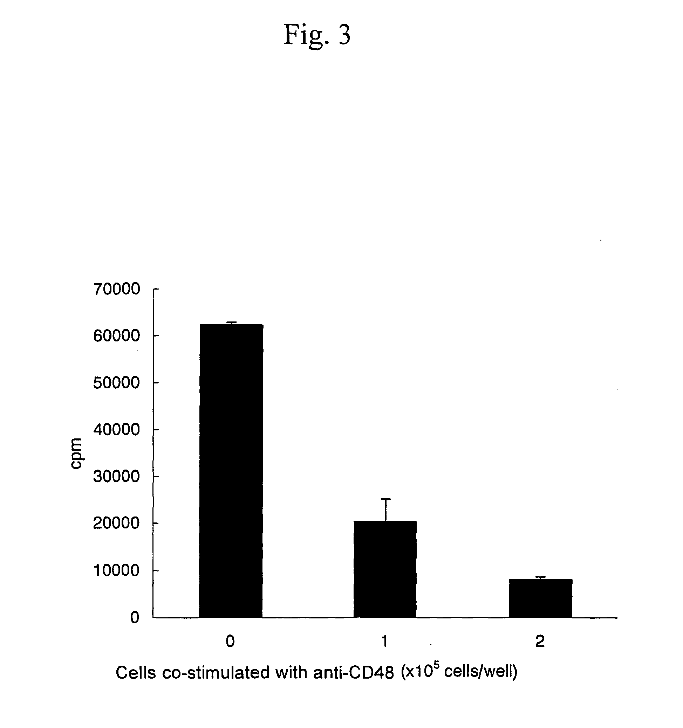

As shown in Fig. 3, the cells co-stimulated with the anti-CD48 antibody suppressed the proliferation of CD4+ T cells due to stimulation with the anti-CD3 antibody, depending on the number of the co-stimulated cells added. It was revealed that regulatory T cells are induced by co-stimulation with the anti-CD48 antibody.

All publications, patents, and patent applications cited herein are incorporated herein by reference in their entirety.

## Claims

1. A method for inducing the differentiation of a regulatory T cell and/or promoting the proliferation of a regulatory T cell, which comprises stimulating a GPI-anchored protein existing on the surface of an immunocyte other than CD52 with an agonist of the protein.

2. The method according to claim 1, wherein the GPI-anchored protein is involved in T cell activation.

3. The method according to claim 1, wherein the GPI-anchored protein is selected from the group consisting of CD55, CD59, and CD48.

4. The method according to claim 1, wherein the agonist is an anti-GPI-anchored protein antibody or a fragment thereof.

5. The method according to claim 4, wherein the anti-GPI-anchored protein antibody is a humanized antibody or a human antibody.

6. The method according to any one of claims 1 to 5, which further comprises stimulating CD3 existing on the surface of an immunocyte with a CD3 agonist.

7. The method according to claim 6, wherein the CD3 agonist is an anti-CD3 antibody or a fragment thereof.

8. The method according to claim 7, wherein the anti-CD3 antibody is a humanized antibody or a human antibody.

9. The method according to any one of claims 1 to 8, wherein the immunocyte is a T cell.

10. The method according to claim 9, wherein the immunocyte is a peripheral blood mononuclear cell.

11. The method according to any one of claims 1 to 10, wherein stimulation of the immunocyte with the CD3 agonist and stimulation of the immunocyte with the GPI-anchored protein agonist are performed *ex vivo.*

12. The method according to any one of claims 1 to 10, wherein stimulation of the immunocyte with the CD3 agonist and stimulation of the immunocyte with the GPI-anchored protein agonist are performed *in vivo.*

13. A regulatory T cell, which is obtained by inducing differentiation and/or promoting proliferation by the method according to any one of claims 1 to 10.

14. A cell-containing product, which contains a regulatory T cell that is obtained by inducing differentiation and/or promoting proliferation by the method according to claims 1 to 10.

15. A pharmaceutical composition for immunosuppression, which contains a regulatory T cell that is obtained by inducing differentiation and/or promoting proliferation by the method according to any one of claims 1 to 10.

16. The pharmaceutical composition according to claim 15 for preventing or treating autoimmune diseases, allergic diseases, or immune responses to transplantation.

17. A method for preparing a humanized antibody or an antibody against a GPI-anchored protein other than CD52, which is a drug having effects of inducing the differentiation of a regulatory T cell and/or promoting the proliferation of a regulatory T cell.

18. The method according to claim 17, wherein the GPI-anchored protein is selected from the group consisting of CD55, CD59, and CD48.

19. A method for screening for a drug having the effects of inducing the differentiation of and/or promoting the proliferation of a regulatory T cell using as an index the interaction with a GPI-anchored protein other than CD52, which comprises causing a cell expressing a GPI-anchored protein other than CD52 to come into contact with a candidate compound and then detecting their interaction or a response of the GPI-anchored protein to stimulation.

20. The method according to claim 19, wherein the GPI-anchored protein is selected from the group consisting of CD55, CD59, and CD48.

21. A regulatory T cell derived from a human immunocyte, which is obtained by stimulating an immunocyte collected from a patient's body or another human's body, specifically by stimulating a GPI-anchored protein existing on the surface of the immunocyte, other than CD52, with an agonist of the protein, so as to induce differentiation into a regulatory T cell and to promote the proliferation of the regulatory T cell.

22. The cell according to claim 21, wherein the GPI-anchored protein is selected from the group consisting of CD55, CD59, and CD48.

23. The regulatory T cell according to claim 21 or 22, which is obtained by, in addition to stimulating the collected immunocyte with the agonist, further stimulating the immunocyte with a CD3 agonist, so as to induce the differentiation into a regulatory T cell and promote the proliferation of the regulatory T cell.

24. A method for producing a regulatory T cell derived from a human immunocyte, which comprises stimulating an immunocyte collected from a patient's body or another human's body, specifically by stimulating a GPI-anchored protein existing on the surface of the immunocyte, other than CD52, with an agonist of the protein, so as to induce the differentiation into a regulatory T cell and promote the proliferation of the regulatory T cell.

25. The method according to claim 24, wherein the GPI-anchored protein is selected from the group consisting of CD55, CD59, and CD48.

26. The method for producing a regulatory T cell according to claim 24 or 25, which further comprises stimulating the collected immunocyte with a CD3 agonist in addition to stimulating the collected immunocyte with the agonist.

27. A pharmaceutical composition for inducing the differentiation of and/or promoting the proliferation of a regulatory T cell, which contains an agonist of a GPI-anchored protein other than CD52 as an active ingredient.

28. The pharmaceutical composition according to claim 27, wherein the GPI-anchored protein is involved in T cell activation.

29. The pharmaceutical composition according to claim 27, wherein the GPI-anchored protein is selected from the group consisting of CD55, CD59, and CD48.

30. The pharmaceutical composition according to claim 27, wherein the agonist is an anti-GPI-anchored protein antibody or a fragment thereof.

31. The pharmaceutical composition according to claim 30, wherein the antibody is a humanized antibody or a human antibody.

32. The pharmaceutical composition according to claim 27, which further contains a CD3 agonist.

33. The pharmaceutical composition according to claim 32, wherein the CD3 agonist is an anti-CD3 antibody or a fragment thereof.

34. The pharmaceutical composition according to claim 33, wherein the anti-CD3 antibody is a humanized antibody or a human antibody.

35. The pharmaceutical composition according to any one of claims 27 to 34 for preventing or treating autoimmune diseases, allergic diseases, or immune responses to transplantation.
